# EUROPÄISCHE PATENTSCHRIFT

(11) **EP 3 528 882 B1**
(45) Veröffentlichungstag und Bekanntmachung des Hinweises auf die Patenterteilung: **16.06.2021**
(21) Anmeldenummer: 17783485.0
(22) Anmeldetag: 11.10.2017
(51) Int. Cl.: A61M 16/20, F16K 7/00, A62B 9/02, A62B 18/10, F16K 15/14

(54) **EXSPIRATIONSVENTIL FÜR EINE BEATMUNGSVORRICHTUNG MIT GERÄUSCHEMISSIONSREDUZIERTER VENTILGESTALTUNG**
EXHALATION VALVE FOR A VENTILATOR APPARATUS WITH A VALVE CONFIGURATION FOR REDUCING NOISE EMISSION
CLAPET D'EXPIRATION POUR DISPOSITIF DE VENTILATION AYANT UNE CONCEPTION DE CLAPET À ÉMISSION DE BRUIT RÉDUITE

(30) Priorität: 24.10.2016 DE 102016220812
(43) Veröffentlichungstag der Anmeldung: 28.08.2019
(73) Patentinhaber: Hamilton Medical AG, 7402 Bonaduz (CH)
(72) Erfinder: HUNGER, Jan, 7440 Andeer (CH)
(74) Vertreter: Ruttensperger Lachnit Trossin Gomoll
(86) Internationale Anmeldenummer: PCT/EP2017/075952
(87) Internationale Veröffentlichungsnummer: WO 2018/077618

(56) Entgegenhaltungen:
- EP-A1- 0 158 553
- EP-A2- 0 144 501
- EP-A2- 0 669 141
- WO-A1-98/41274
- WO-A1-99/59517
- WO-A1-2015/136407
- WO-A2-2009/028938
- US-A- 3 608 574
- US-A- 4 406 302
- US-A- 4 611 591
- US-A- 4 699 137
- US-A- 4 823 828
- US-A- 5 704 348

## Beschreibung

Die vorliegende Erfindung betrifft ein Exspirationsventil für eine Beatmungsvorrichtung zur wenigstens teilweise apparativ unterstützten Beatmung eines Patienten mit den Merkmalen des Oberbegriffs des Anspruchs 1.

Ein derartiges Exspirationsventil ist beispielsweise aus der US 4823828 bekannt.

Weitere Exspirationsventile sind aus den Druckschriften US 3608574, US 4699137, WO 2009/028938 A2, WO 2015/136407 A1, EP 0669141 A2, US 4611591, WO 99/59517 A1, US 5704348, US 4406302, EP 0158553 A1, WO 98/41274 A1, EP 0144501 A2 bekannt. Außerdem sind Exspirationsventile beispielsweise von den Beatmungsgeräten der Anmelderin mit der Produktbezeichnung "C2" oder "C3" bekannt. Das Exspirationsventil dient in einer Beatmungsvorrichtung zur Steuerung des Atemgastransports.

Beatmungsvorrichtungen weisen üblicherweise eine Atemgas-Förderpumpe auf, um Atem-Frischgas zu einem zu beatmenden Patienten zu fördern. Die Beatmungsvorrichtung weist in der Regel ein Inspirationsventil auf, welches eine Förderung von Atem-Frischgas von der Beatmungsvorrichtung weg zum Patienten hin zulässt, in umgekehrter Richtung jedoch sperrt, und weist ein Exspirationsventil auf, welches eine Strömung von Atemgas in Exspirationsströmungsrichtung vom Patienten weg zur Beatmungsvorrichtung hin zulässt, in entgegengesetzter Richtung jedoch sperrt.

Bekannte Exspirationsventile weisen einen tellerartigen Ventilkörper auf, welcher der Endfläche der gehäusefesten Ventil-Teilformation gegenüberliegt und auch auf dieser aufliegen kann, um eine Gasströmung in einer der Exspirationsströmungsrichtung entgegengesetzte Richtung zu sperren. Der der Endfläche in Abheberichtung gegenüberliegende oder an dieser anliegende Teil der Außenfläche des tellerartigen Ventilkörpers ist dann die oben genannte Gegenfläche.

Der Ventilkörper und die gehäusefeste Ventil-Teilformation ergänzen sich funktionell zu einer Ventilformation, die eine Ventilfunktion im Sinne einer Strömungssteuerung wahrnehmen kann.

Bei den bekannten Exspirationsventilen wird der tellerförmige Ventilkörper während eines Exspirationsvorgangs in Exspirationsströmungsrichtung angeströmt, so dass auf der Anströmseite des Ventilkörpers der Atemgasdruck ansteigt, während auf der der Anströmseite entgegengesetzten Schattenseite des Ventilkörpers weiterhin Umgebungsdruck herrscht. Dann, wenn der Druckanstieg auf der Anströmseite die Vorspannkraft der Vorspanneinrichtung überwindet, wird der Ventilkörper von der Endfläche weg bewegt, so dass ein zwischen Ventilkörper bzw. dessen Gegenfläche und der Endfläche entstehender oder vorhandener Ringspalt vergrößert wird. Dadurch nimmt der Strömungswiderstand des Exspirationsventils in Exspirationsströmungsrichtung stark ab, so dass verbrauchtes Atemgas in Exspirationsströmungsrichtung ohne großen Widerstand vom Patienten weg strömen kann.

Der Ventilkörper des bekannten Exspirationsventils lenkt als tellerförmiger Ventilkörper die auf ihn auftreffende Atemgasströmung um etwa 90° um, so dass das Atemgas bei ausreichend von der Endfläche entferntem Ventilkörper radial durch den oben beschriebenen Ringspalt strömt. Dabei kann es im Bereich zwischen Endfläche und Ventilkörper zu periodischer Wirbelbildung und zu lokalen Strömungsablösungen kommen, was Druckschwankungen im Exspirationsventil bewirken kann. Diese Druckschwankungen sind in manchen Betriebszuständen außerhalb des Exspirationsventils und außerhalb der Beatmungsvorrichtung unerwünschterweise akustisch wahrnehmbar. Je nach Periodizität der Druckschwankungen können sich diese durch ein Pfeifen oder Rauschen äußern.

Es ist daher Aufgabe der vorliegenden Erfindung, das gattungsgemäße Exspirationsventil derart weiterzubilden, dass es ohne Einschränkung seiner Leistungsfähigkeit als Exspirationsventil im bestimmungsgemäßem Betrieb weniger Geräusche emittiert.

Diese Aufgabe wird erfindungsgemäß gelöst durch ein Exspirationsventil mit allen Merkmalen des Anspruchs 1. Bei einem solchen Exspirationsventil weist der Ventilkörper eine Schürze auf, welche, - bei Betrachtung des Exspirationsventils in einem durch bestimmungsgemäße Atemströmung unbelasteten Bezugszustand - die Gegenfläche und die Endfläche umgebend, sich in einer Umfangsrichtung erstreckt und welche im Bezugszustand entgegen der Abheberichtung in Richtung von der Gegenfläche weg axial über die Endfläche hinaus absteht, wobei radial zwischen der Schürze und einem die Endfläche aufweisenden Endabschnitt der Ventil-Teilformation ein Ringspaltraum vorgesehen ist.

Da das erfindungsgemäße Exspirationsventil bevorzugt bestimmungsgemäß lösbar bzw. abnehmbar an einer Beatmungsvorrichtung vorgesehen ist, wird das Exspirationsventil in einem Bezugszustand diskutiert, in welchem es durch Atemströmung unbelastet ist. Dies entspricht etwa einem Bezugszustand, in welchem ein von einer Beatmungsvorrichtung abgenommenes Exspirationsventil in einem Regal oder auf einer Werkbank zur weiteren Verwendung bereit liegt.

Durch die Schürze, die die Gegenfläche und die Endfläche im Bezugszustand umgibt, also bezogen auf eine den Ventilkörper in Abheberichtung zentral durchsetzende Körperachse radial außen umgibt, wird auf den Ventilkörper auftreffende Exspirationsströmung nicht mehr nur radial nach außen umgelenkt, sondern wird an der Schürze zwangsweise in eine Strömungsrichtung mit einer Komponente entgegen der Exspirationsströmung umgelenkt, die dann in der der anströmenden Exspirationsströmung entgegengesetzten Richtung durch den zwischen der Schürze und der Ventil-Teilformation gebildeten Ringspaltraum strömen muss.

Damit wird durch das Vorsehen der Schürze die Exspirationsströmung entlang eines längeren Wegs als im Stand der Technik über die Endfläche hinaus an der Ventil-Teilformation und am Ventilkörper vorbei geführt. Während im Stand der Technik die Exspirationsströmung, nachdem sie die Endfläche passiert hat, im Wesentlichen als Freistrahl strömt, ist die Exspirationsströmung im erfindungsgemäßen Zustand auch noch nach Passieren der Endfläche körperlich durch die Schürze und durch die Ventil-Teilformation geführt, die beide den genannten Ringspaltraum begrenzen.

Nachzutragen ist, dass mit einer eingangs genannten, durch die Durchgangsbahn definierten lokalen Koordinatenrichtung (axial, radial, Umfangsrichtung) ausgesagt sein soll, dass sich durchgangsbahnbezogene benennungsgleiche Koordinatenrichtungen in einem absoluten Koordinatensystem betrachtet abhängig vom Ort längs der Durchgangsbahn unterscheiden können, etwa weil die Durchgangsbahn einen gekrümmten oder abgewinkelten Verlauf hat.

Der Ringspaltraum umgibt bevorzugt radial außen jenen mit Bezug auf die Exspirationsströmungsrichtung stromaufwärts vorder Gegenfläche gelegenen Abschnitt des Strömungsdurchgangs, in welchem die Exspirationsströmung zum Ventilkörper hingeleitet wird.

Die Schürze kann sich ausgehend von einem die Gegenfläche aufweisenden Abschnitt des Ventilkörpers entgegen der Abheberichtung beliebig weit von dem Abschnitt weg erstrecken, so dass der Ringspaltraum, wenngleich mit unterschiedlichen, parallel zur Abheberichtung zu messenden Spaltraumhöhen, unabhängig von der Betriebsposition des Ventilkörpers über den gesamten bestimmungsgemäßen Bewegungsbereich des Ventilkörpers in Abheberichtung bestehen kann. Die Schürze kann jedoch alternativ entgegen der Abheberichtung kürzer bemessen sein als der maximale Abhebeweg des Ventilkörpers ausgehend von seiner Bezugsposition in Richtung von der Endfläche weg, so dass dann, wenn der Ventilkörper während eines Exspirationsvorgangs einen vorbestimmten Abhebe-Schwellenweg überschreitet, der Schürze radial innen kein Abschnitt der Ventil-Teilformation mehr gegenüberliegt und somit kein Ringspaltraum mehr besteht.

Durch das Vorsehen der Schürze und durch die damit verbundene Änderung der Strömungsverhältnisse am Ventilkörper werden während eines Exspirationsvorgangs stabilere Strömungsverhältnisse im Bereich von Endfläche und Gegenfläche erzielt als am schürzenlosen Ventilkörper des Standes der Technik. Die stabileren Strömungsverhältnisse führen zu weniger bis überhaupt keinen Druckschwankungen mehr, so dass auch die im Stand der Technik mit wiederholter oder periodischer Wirbelbildung und Druckschwankung einhergehenden Geräuschemissionen erheblich reduziert sind.

Die Reduzierung der Geräuschemissionen des Exspirationsventils sind in einem betriebsmäßig relevanten Volumenstrombereich von etwa 15 Litern pro Minute besonders ausgeprägt.

Eine noch wirkungsvollere Minderung der betriebsmäßig entstehenden Geräuschemissionen wird erfindungsgemäß dadurch erreicht, dass ein parallel zur Abheberichtung zu messender Abstand eines von der Gegenfläche fernen Schürzenrandes von der Gegenfläche wenigstens umfangsabschnittsweise abhängig von der jeweiligen Position in Umfangsrichtung unterschiedlich groß ist. In diesem Falle kragt die Schürze längs ihres Umfangs unterschiedlich weit von einem die Gegenfläche aufweisenden Abschnitt des Ventilkörpers aus. Bevorzugt ändert sich der Abstand bzw. die Auskraglänge der Schürze längs ihrer Erstreckung in Umfangsrichtung um die zur Abheberichtung parallele Körperachse des Ventilkörpers herum periodisch, so dass längs der Umfangserstreckung der Schürze zwar unterschiedliche, jedoch periodisch wiederkehrende Strömungsverhältnisse auftreten können, was zu einer zusätzlichen Stabilisierung der Exspirationsströmung während eines Exspirationsorgangs im Bereich zwischen Endfläche und Gegenfläche beitragen kann. Folglich weist der von der Gegenfläche ferne Schürzenrand bevorzugt eine in Umfangsrichtung verlaufende Wellenform auf. Dabei ist mit Wellenform jegliche periodische Änderung des oben genannten Abstands bzw. der Auskragtiefe bezeichnet.

Die Wellenform kann eine geradlinige Berandung aufweisen und kann beispielsweise als Sägezahn- oder Dreieckswellenprofil ausgestaltet sein, wobei aufgrund bisher erzielter Testergebnisse ein Dreiecksprofil mit einer Folge von in Umfangsrichtung aufeinander folgenden gleichschenkligen Dreiecken bevorzugt ist. Bevorzugt folgen identische gleichschenklige Dreiecke in Umfangsrichtung aufeinander.

Ebenso ist denkbar, dass der von der Gegenfläche ferne freie Seitenrand als geradlinig berandeter Seitenrand eine Rechteck-Wellenform aufweist.

Alternativ oder zusätzlich kann der Seitenrand eine Teilkreis-Wellenform oder allgemein eine sinusförmige Wellenform aufweisen, so dass der Schürzenrand in diesem Fall durch eine krummlinige, bevorzugt ecken- und knickfreie Randlinie definiert ist.

Auch für die Rechteck-Wellenform und für die Teilreis-Wellenform ebenso wie für die sinusförmige Wellenform des Schürzenrandes ist bevorzugt, dass der Schürzenrand durch eine Folge von in Umfangsrichtung aufeinanderfolgenden identischen Randelementen gebildet ist.

Dabei ist zur Erzielung möglichst einheitlicher sich periodisch ändernder Strömungsverhältnisse längs des Umfangs der Schürze bevorzugt, dass wenigstens ein Teil, vorzugsweise alle, der entgegen der Abheberichtung von der Gegenfläche fernstgelegenen Extrempunkte der Wellenberge auf einer Ebene oder/und dass wenigstens ein Teil, vorzugsweise alle, der entgegen der Abheberichtung der Gegenfläche nächstgelegenen Extrempunkte der Wellentäler auf einer Ebene gelegen sind, insbesondere auf einer Ebene, welche zum Verlauf der Durchgangsbahn am Durchstoßpunkt der Ebene oder/und zur Abheberichtung orthogonal ist. Dies ist vor allem dann bevorzugt, wenn die Exspirationsströmungsrichtung unmittelbar am Ventilkörper mit der Abheberichtung im Wesentlichen identisch ist. Liegen die Extrempunkte sowohl der Wellenberge wie auch der Wellentäler jeweils auf einer Ebene existieren zwei, vorzugsweise parallele, Extrempunkte-Ebenen, die im Abstand der Wellenamplitude voneinander entfernt angeordnet sind.

Bevorzugt ist die Gegenfläche oder/und die Endfläche in einer Ebene gelegen. Diese Ebene ist bevorzugt orthogonal zur Abheberichtung orientiert.

Dabei ist mit "Ebene" keine unendlich dünne Ebene im mathematischen Sinne gemeint. Als "in einer Ebene gelegen" soll die Gegenfläche oder/und die Endfläche vielmehr bereits dann angesehen werden, wenn wenigstens ein axialer Rand der Gegenfläche oder der Endfläche, vorzugsweise beide die jeweiligen Flächen axial längs der Abheberichtung eingrenzenden Ränder, eben sind. Die Gegenfläche oder/- und die Endfläche können beispielsweise eine Mantelfläche eines Kegelstumpfs beschreiben.

Mit in einer Ebene gelegenen Flächen aus Gegenfläche und Endfläche lassen sich hervorragende Dichtungswirkungen bei gleichzeitiger Zentrierungswirkung von Endfläche und Gegenfläche im Zusammenspiel miteinander sicherstellen, ohne dass hierfür unnötig viel Bauraum bereitgestellt werden muss.

Bevorzugt sind die Gegenfläche oder/und die Endfläche eben. Dann kann das Exspirationsventil mit besonders kurzen Abmessungen ausgebildet werden. Eine Zentrierwirkung zwischen Gegenfläche und Endfläche entfällt dann. Eine Zentrierwirkung kann jedoch durch die oben genannte Vorspanneinrichtung bewirkt werden.

Eine ebene Gegenfläche oder/und Endfläche ist dabei eine Sonderform einer in einer Ebene gelegen Gegenfläche oder/und Endfläche. Bevorzugt ist die Ebene, in der die Gegenfläche oder/und die Endfläche gelegen ist, parallel zu der Ebene, auf welcher die fernst- oder/und nächstgelegenen Extrempunkte der Wellenberge bzw. Wellentäler eines wellenförmigen Schürzenrandes gelegen sind.

Die Endfläche kann an einem Längsende eines rohrförmigen Abschnitts des Strömungsdurchgangs gelegen oder ausgebildet sein. Der rohrförmige Abschnitt des Strömungsdurchgangs bildet dann den oben genannten Endabschnitt der Ventil-Teilformation. Bevorzugt ist der Strömungsdurchgang auf der in Exspirationsströmungsrichtung zum Ventilkörper hinweisenden Seite durch ein Atemrohr, insbesondere gerades Atemrohr, gebildet, welches Teil des Ventilgehäuses sein kann. Dann fällt eine Rohrachse des Atemrohrs bevorzugt über die gesamte Rohrstrecke, jedoch wenigstens im Annäherungsbereich an den Ventilkörper mit einer Parallelen zur Abheberichtung zusammen. Die Exspirationsströmungsrichtung verläuft dann längs der Atemrohrachse. Die Atemrohrachse ist dabei kollinear mit der oben beschriebenen Ventilkörperachse. Die Rohrachse ist Teil der Durchgangsbahn.

Die Schürze umgibt dann im Bezugszustand einen sich längs der Durchgangsbahn - die bei Verwendung eines Atemrohrs im Bereich des Atemrohrs ebenfalls mit der Atemrohrachse zusammenfällt - und in Umfangsrichtung um diesen herum erstreckenden Endbereich des rohrförmigen Abschnitts. Somit bildet der die Endfläche aufweisende Endbereich des rohrförmigen Abschnitts, insbesondere des Atemrohrs, zusammen mit der ihn umgebenden Schürze den eingangs genannten Ringspaltraum. Längs der Atemrohrachse bzw. längs der Durchgangsbahn überlappen sich somit Schürze und das Längsende des rohrförmigen Abschnitts axial.

Zur verbesserten Strömungsführung kann ein radial äußerer Bereich des Längsendes des rohrförmigen Abschnitts angefast sein. In diesem Falle ist die radiale Erstreckung der Endfläche wegen der Fase am Längsende des rohrförmigen Abschnitts in radialer Richtung kürzer als die radiale Abmessung des ungefast gedachten rohrförmigen Abschnitts. Dann kann zwischen der Endfläche und der diese radial außen umgebenden Schürze mit der Anfasung ein in radiale Richtung wirkender Expansionsraum bereitgestellt sein, in den die vom Ventilkörper in radiale Richtung abgelenkte Exspirationsströmung nach Vorbeistreichen an der Endfläche hinein entspannen kann.

Damit die Schürze ihre oben beschriebene Wirkung der Strömungsführung auch bei angefastem Abschnitt möglichst gut entfalten kann, reicht die Schütze wenigstens im Bezugszustand entgegen der Abheberichtung über das axial weiter von der Endfläche entfernt gelegene Fasenende hinaus. Somit kann in einem Bereich zwischen dem weiter von der Endfläche entfernt gelegenen Fasenende und den freien Schürzenrand ein Ringspaltraum bestehen. Es kann jedoch daran gedacht sein, bei der oben beschriebenen bevorzugten wellenförmigen Ausgestaltung des freien Schürzenrandes die Wellentäler, also die näher bei der Gegenfläche gelegenen Randbereiche, von der Gegenfläche weniger weit entfernt anzuordnen als das weiter von der Endfläche entfernt gelegene Fasenende. Die Wellenberge können jedoch entgegen der Abheberichtung über das betreffende Fasenende hinausreichen. In diesem Falle können ausgehend von dem durch die Anfasung gebildeten radialen Expansionsraum Entweichungsöffnungen gebildet sein, welche aufgrund der Wellenform periodisch in Umfangsrichtung um den Endbereich des rohrförmigen Abschnitts herum gelegen sein können.

Konstruktiv hat es sich im Hinblick auf eine Minderung des Betriebsgeräusches des Exspirationsventils als vorteilhaft herausgestellt, wenn sich im Bezugszustand der radiale Abstand des näher an der Endfläche gelegenen Fasenendes von einer nach radial innen weisenden Wandung der Schürze einerseits und die Überlappungstiefe von Schürze und rohrförmigem Abschnitt parallel zur Abheberichtung andererseits um nicht mehr als 20 %, vorzugsweise um nicht mehr als 10 % unterscheiden, besonders bevorzugt gleich sind.

Ebenso ist es aus dem gleichen Grunde konstruktiv vorteilhaft, wenn sich im Bezugszustand die radiale Abmessung des Ringspaltraums einerseits und die radiale Dicke der Schürze andererseits in einem den von der Gegenfläche fernliegenden Schürzenrand enthaltenden Endbereich der Schürze um nicht mehr als 20 %, vorzugsweise um nicht mehr als 10 % unterscheiden, besonders bevorzugt gleich sind. Ein kurzes Exspirationsventil kann dadurch erhalten werden, dass ein Teil des Strömungsdurchgangs durch ein Atemrohr und einen das Atemrohr, vorzugsweise koaxial, umgebenden Ringkanal gebildet ist, wobei der Ringspalt zwischen Endfläche und Gegenfläche strömungsmechanisch in Exspirationsströmungsrichtung zwischen dem Atemrohr und dem Ringkanal gebildet ist. Die längs der Atemrohrachse kurze Abmessung des Exspirationsventils wird zwar auf Kosten eines wegen des Ringkanals größeren Durchmessers erhalten. Jedoch erfordert der Ringkanal nur eine geringe Durchmesservergrößerung des Ventilgehäuses, um einen Ringkanal mit gleichem Strömungsquerschnitt wie das vom Ringkanal umgebene Atemrohr bereitzustellen.

Ganz grundsätzlich kann der Ventilkörper ein beliebig ausgestalteter Ventilkörper sein. Es kann der Ventilkörper beispielsweise eine Ventilkugel sein. Bevorzugt ist der Ventilkörper jedoch ein in Exspirationsventilen bereits bewährter Ventilkörper mit einem im Wesentlichen ebenen Tellerabschnitt, welcher die Gegenfläche aufweist. Ein solcher Ventilkörper trägt weiter zu einer kurzen Bauweise des Exspirationsventils bei. Vorzugsweise ist der Tellerabschnitt zentral am Ventilkörper vorgehsehen.

Die Vorspanneinrichtung kann durch eine beliebige, eine Vorspannkraft ausübende Vorrichtung gebildet sein, einschließlich beispielsweise einer oder mehrerer Schraubendruckfedern. Eine baulich kurze Vorspanneinrichtung, welche gleichzeitig nicht nur den Ventilkörper bzw. dessen Tellerabschnitt zur Endfläche hin vorspannt, sondern bezüglich derselben auch zentrieren kann, ist eine Membranfeder, welche den Tellerabschnitt radial außen umgibt und den Tellerabschnitt mit einem diesen in radialem Abstand umgebenden Befestigungsabschnitt des Ventilkörpers verbindet.

Besonders bevorzugt spannt die Vorspanneinrichtung die Gegenfläche nicht nur zur Endfläche hin vor, sondern spannt die Gegenfläche oder/und einen die Gegenfläche aufweisenden Ventilabschnitt in einer zur Abheberichtung orthogonalen Ebene in eine vorbestimmte Ruhestellung vor. Dies kann durch eine in Umfangsrichtung um die Gegenfläche herum angeordnete und in unterschiedlichen radialen Richtungen zur Mitte der Durchgangsbahn an deren Durchstoßort durch einen die Gegenfläche aufweisenden Ventilkörperabschnitt wirkende Vorspanneinrichtung realisiert sein. Die oben genannte, den Tellerabschnitt bzw. einen die Gegenfläche aufweisenden Ventilkörperabschnitt radial außenumgebende Membranfeder kann al solche Vorspanneinrichtung verwendet werden. So kann die Vorspanneinrichtung die Gegenfläche relativ zur Durchgangsbahn zentrieren.

Zusätzlich oder alternativ kann die Vorspanneinrichtung die Gegenfläche bzw. einen die Gegenfläche aufweisenden Ventilkörperabschnitt, etwa der oben genannte Tellerabschnitt, während einer Abhebe- und Rückstellbewegung in beziehungsweise entgegen der Abheberichtung führen. Die Führung muss dabei keine exakte Führung im Sinne einer Spurbindung sein. Es reicht vorliegend aus, wenn die Vorspanneinrichtung eine Abweichung der Bewegung der Gegenfläche von der Abheberichtung begrenzt, so dass sich die Gegenfläche im bestimmungsgemäßen Betrieb von einem idealen Bewegungspfad längs der Abheberichtung nur um ein durch die Vorspanneinrichtung bestimmtes, nicht überschreitbares Maximalmaß abweichen kann.

Der Befestigungsabschnitt kann zur bevorzugt formschlüssigen Verbindung mit dem Ventilgehäuse ausgebildet sein, insbesondere mit einem den Ringkanal begrenzenden Abschnitt des Ventilgehäuses. Dann kann der Befestigungsabschnitt an einem den Ringkanal radial außen umgebenden Gehäuseabschnitt des Ventilgehäuses festgelegt sein, die Membranfeder kann den radialen Abstand zu einem radial weiter innen gelegenen Atemrohr überspannen und der Tellerabschnitt kann einem Endabschnitt des Atemrohrs gegenüberliegen. Bevorzugt ist der Ventilkörper zur Erleichterung seiner Montage bezüglich einer den Tellerabschnitt orthogonal zur Tellerebene durchsetzenden Ventilkörperachse rotationssymmetrisch ausgebildet.

Der Ventilkörper ist bevorzugt aus einem elastomeren Material gebildet, wie beispielsweise Silikon, Kautschuk und dergleichen.

Die Gegenfläche kann im Bezugszustand auf der Endfläche aufliegen oder mit geringem Abstand von dieser angeordnet sein, wobei "gering" in Bezug auf den betriebsmäßig maximalen Hub der Gegenfläche in Abheberichtung während eines Exspirationsvorgangs zu setzen ist. Vorzugsweise überschreitet ein Abstand der Gegenfläche von der Endfläche im Bezugszustand 10%, oder bevorzugt 5% des betriebsmäßig maximal möglichen Abstands der Gegenfläche von der Endfläche während eines Exspirationsvorgangs nicht.

Bevorzugt ist der Ventilkörper einstückig ausgebildet, so dass der Befestigungsabschnitt, die Membranfeder und der Tellerabschnitt ein monolitisches Bauteil bilden. Zur Verstärkung des Tellerabschnitts kann an diesem ein Verstärkungsbauteil vorgesehen sein, etwa eine Metallscheibe oder/und eine Keramikscheibe. Um den Tellerabschnitt vor äußeren Einflüssen und Belastungen schützen zu können, liegt das Verstärkungsbauteil bevorzugt im am Tellerabschnitt angebrachten Zustand frei, und zwar besonders bevorzugt auf der von der Endfläche weg weisenden, also nicht von der Exspirationsströmung angeströmten Seite.

Um gegebenenfalls unabhängig von einer Atemaktivität des Patienten das Exspirationsventil steuern zu können, kann mit dem Ventilgehäuse ein Aktuator verbunden sein, von welchem ein Stellglied wenigstens zur Verlagerung der Gegenfläche entgegen der Abheberichtung mit dem Ventilkörper zusammenwirkt. Das Stellglied kann ein mechanisches Stellglied sein, etwa ein Stößel, ein Lenker oder ein Verbindungsglied, welches mit dem Tellerabschnitt, insbesondere dort mit dem Verstärkungsbauteil zusammenwirkt oder sogar zur gemeinsamen Bewegung mechanisch gekoppelt ist. Das Stellglied kann jedoch im Falle eines magnetisierten Verstärkungsbauteils auch ein Anker oder Ankerabschnitt eines wahlweise bestrombaren Elektromagneten sein. Beispielsweise kann ein Aktuatorgehäuse oder allgemein eine Akuator-Trägerstruktur mit dem Ventilgehäuse verbunden sein, wobei das Stellglied des Aktuators relativ zum Aktuatorgehäuse bzw. zur Akuator-Trägerstruktur beweglich ist, um mit dem Ventilkörper zusammenzuwirken.

Der Aktuator soll auch dann als mit dem Ventilgehäuse verbunden gelten, wenn der Aktuator, etwa ein Aktuatorgehäuse oder allgemein eine Akuator-Trägerstruktur, mittels Zwischenanordnung eines oder mehrerer Bauteile fest mit dem Ventilgehäuse verbunden ist.

Zum Schließen des Exspirationsventils durch den Aktuator kann es ausreichen, wenn das Stellglied mit dem Ventilkörper alleine zur Verlagerung der Gegenfläche entgegen der Abheberichtung zusammenwirkt. Hierfür bedarf es nicht notwendigerweise einer dauerhaften Kopplung des Stellglieds mit dem Ventilkörper. Es kann dann ausreichen, dass ein Stößel zum Ventilkörper hin ausgefahren wird, mit diesem in Anlageeingriff gelangt und diesen mit seiner Gegenfläche gegen die Endfläche drückt. Das Stellglied kann in Abheberichtung vom Tellerabschnitt, insbesondere von dem Verstärkungsbauteil abhebbar sein, um eine allein durch die Exspirationsströmung bewirkte Abhebebewegung der Gegenfläche zu ermöglichen.

Der vorübergehend herstellbare Anlageeingriff zwischen Stellglied und Tellerabschnitt, insbesondere mit dem freiliegenden Abschnitt des Verstärkungsbauteils, ist eine Ausgestaltung einer Koppelbarkeit des Stellglieds des Aktuators mit dem Ventilkörper. Um den Ventilkörper sowohl entgegen der Abheberichtung als auch in Abheberichtung durch den Aktuator bewegen zu können, kann dieser mit dem Ventilkörper, insbesondere mit dem Verstärkungsbauteil, magnetisch oder mechanisch gekoppelt oder koppelbar sein, beispielsweise durch eine mechanische Verrastung oder durch eine magnetische Kopplung oder Koppelbarkeit.

Ist die mechanische Verrastung überwindbar ausgestaltet, kann diese auch einfach wieder bedarfsweise gelöst werden.

Die vorliegende Erfindung betrifft weiter eine Beatmungsvorrichtung zur wenigstens teilweise apparativ unterstützten Beatmung eines Patienten, welche eine Atemgas-Förderpumpe aufweist, um einen Atemgas-Förderstrom zu bewirken, welche weiter ein Inspirationsventil aufweist, und welche ein Exspirationsventil aufweist, wie es oben beschrieben ist. Ein Gehäuse der Beatmungsvorrichtung kann das Bauteil oder kann eines der Bauteile sein, mittels dem bzw. denen der Aktuator mit dem Ventilgehäuse verbunden ist. Beispielsweise kann sowohl das Ventilgehäuse als auch ein Aktuatorgehäuse fest mit dem Gehäuse der Beatmungsvorrichtung verbunden sein. Dann ist auch das Aktuatorgehäuse mit dem Ventilgehäuse verbunden.

Da die Weiterbildung des eingangs genannten gattungsgemäßen Exspirationsventils zur Lösung der oben genannten Aufgabe im Wesentlichen am Ventilkörper realisiert ist, betrifft die vorliegende Erfindung auch einen Ventilkörper für ein Exspirationsventil mit allen Merkmalen des Anspruchs 17, insbesondere für ein gemäß der obigen Beschreibung ausgebildetes Exspirationsventil. Ein solcher Ventilkörper umfasst eine zur Anlage an einer (oben als "Endfläche" bezeichneten) Ventilsitzfläche ausgebildete (oben als "Gegenfläche" bezeichneten) Anlagefläche, welche längs einer Bewegungsachse in und entgegen einer Abheberichtung bewegbar ist, wobei die Anlagefläche mit der Bewegungsachse einen, vorzugsweise rechten, Winkel einschließt, und wobei der Ventilkörper eine Schürze aufweist, welche die Anlagefläche bezüglich der Bewegungsachse radial außen umgibt und welche ausgehend von einem die Anlagefläche aufweisenden Ventilkörperabschnitt bezüglich der Bewegungsachse axial von dem Ventilkörperabschnitt auskragt und dabei die Anlagefläche axial überragt, insbesondere längs ihres gesamten Umfangs um die Bewegungsachse herum überragt.

Der Ventilkörperabschnitt kann der oben genannte Tellerabschnitt sein. Die Bewegungsachse kann die oben genannte Ventilkörperachse des Ventilkörpers sein oder/- und kann richtungsmäßig mit der oben genannten Durchgangsbahn im Bereich der Anlagefläche/Gegenfläche zusammenfallen.

Oben beschriebene Weiterbildungen des Exspirationsventils, die alleine den Ventilkörper betreffen, sind auch vorteilhafte Weiterbildungen des erfindungsgemäßen Ventilkörpers und umgekehrt. Dies gilt insbesondere für die wellenförmige Ausbildung des freien Schürzenrandes am von der Anlagefläche/Gegenfläche ferner gelegenen axialen Längsende der Schürze. An dem Längsende der Schürze ist bevorzugt kein Teil der Anlagefläche und auch keine weitere Anlagefläche ausgebildet.

Bevorzugt weist der Ventilkörper eine Vorspanneinrichtung auf, die mit dem die Anlagefläche/Gegenfläche aufweisenden Ventilkörperabschnitt verbunden ist, insbesondere einstückig verbunden ist, und einer Verlagerung des Ventilkörperabschnitts sowohl längs der Bewegungsachse als auch orthogonal dazu eine Vorspannkraft entgegensetzt. Bevorzugt ist die Vorspanneinrichtung derart, dass die von ihr auf den Ventilkörperabschnitt ausgeübte Vorspannkraft mit zunehmendem Verlagerungsbetrag des Ventilkörperabschnitts ausgehend von seiner unbelasteten Ruheposition ansteigt.

Weiter bevorzugt weist der Ventilkörper einen Befestigungsabschnitt auf, mit welchem der Ventilkörper an einem Ventilgehäuse oder allgemein einer Ventil-Grundstruktur befestigbar ist. Der Befestigungsabschnitt ist bevorzugt einstückig mit dem Ventilkörperabschnitt ausgebildet, besonders bevorzugt auch einstückig mit der Vorspanneinrichtung. Der Befestigungsabschnitt weist vorteilhafterweise eine Formschlussformation zur Befestigung des Ventilkörpers auf.

Die Vorspanneinrichtung ist bevorzugt als Membranfeder ausgebildet. Sie umgibt zur Bereitstellung identischer Vorspannkräfte unabhängig um Umfangsort bevorzugt den Ventilkörperabschnitt um die Bewegungsachse herum vollständig.

Die vorliegende Erfindung wird nachfolgend anhand der beiliegenden Zeichnungen näher dargestellt. Es stellt dar:
- Fig. 1: eine Längsschnittansicht eines beispielhaften Exspirationsventils der vorliegenden Anmeldung,
- Fig. 2: eine längsgeschnittene perspektivische Ansicht eines erfindungsgemäßen Exspirationsventils,
- Fig. 3: eine Längsschnittansicht durch den Ventilkörper des Exspirationsventils der Fig. 1 ,
- Fig. 4: eine Detailansicht des Ausschnitts IV von Fig. 3,
- Fig. 5: eine Detailansicht des Ausschnitts V von Fig. 1,
- Fig. 6: eine Aufrissansicht des erfindungsgemäßen Ventilkörpers des Exspirationsventils von Fig. 2,
- Fig. 7: eine erste alternative Formation des erfindungsgemäßen freien Schürzenrandes und
- Fig. 8: eine zweite alternative Formation des erfindungsgemäßen freien Schürzenrandes.

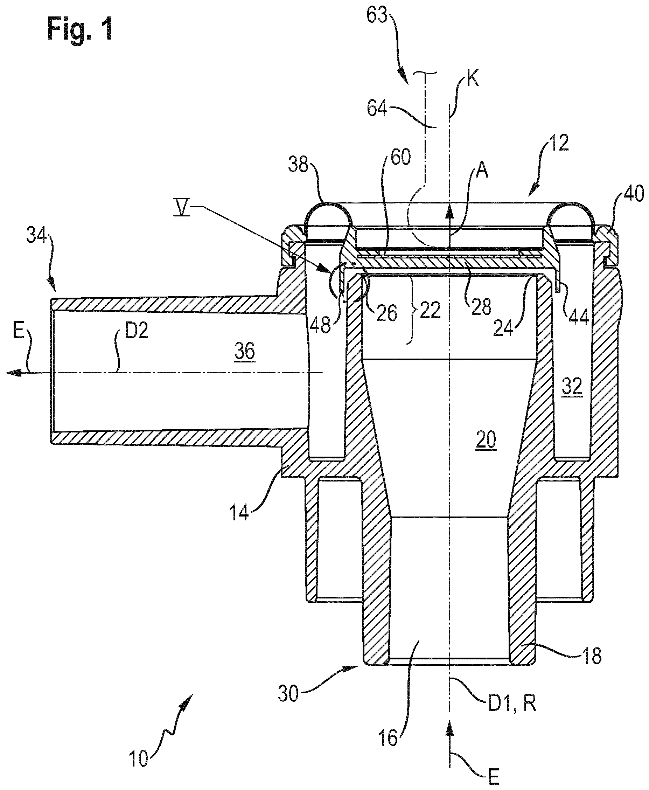

In Fig. 1 ist ein Beispiel eines Exspirationsventils allgemein mit 10 bezeichnet und im Längsschnitt dargestellt. Die Schnittebene enthält die Durchgangsbahn D, die in dem gezeigten Beispiel zwei Abschnitte aufweist, nämlich den Abschnitt D1 in Exspirationsströmungsrichtung E stromaufwärts eines Ventilkörpers 12 und den Abschnitt D2 stromabwärts des Ventilkörpers 12.

Der Ventilkörper 12 ist an einem Ventilgehäuse 14 gehaltert, welches bevorzugt einstückig, beispielsweise im Spritzgussverfahren, hergestellt ist.

In dem Ventilgehäuse oder Gehäuse 14 ist ein Strömungsdurchgang 16 ausgebildet, welcher sich längs der Durchgangsbahnabschnitte D1 und D2 erstreckt.

Das Gehäuse 14 weist ein integral daran ausgebildetes Atemrohr 18 auf, welches geradlinig längs des Durchgangsbahnabschnitts D1 unter lokaler Aufweitung in Exspirationsströmungsrichtung E zum Ventilkörper 12 hin verläuft. Der Durchgangsbahnabschnitt D1 fällt daher mit der Rohrachse R des Atemrohrs 18 zusammen. Das Atemgasrohr 18 bildet damit einen stromaufwärtigen Strömungsdurchgangsabschnitt 20 des Strömungsdurchgangs 16. An einem dem Ventilkörper 12 nächstgelegenen Endabschnitt 22 des Atemrohrs 18 ist eine stirnseitig an dem Atemrohr 18 um den Strömungsdurchgang 16 in diesem Bereich umlaufende Endfläche 24 ausgebildet, der eine Gegenfläche 26 eines im Wesentlichen eben ausgebildeten tellerartigen Abschnitts 28 des Ventilkörpers 12 gegenüberliegt.

In dem in den Figuren dargestellten Bezugszustand befindet sich die Gegenfläche 26 in einem geringfügigen Spaltabstand von der Endfläche 24 entfernt. Sobald jedoch ein am proximalen Ende 30 des Exspirationsventils 10 angeschlossener Patient einatmet, würde durch den so entstehenden Druckunterschied am Ventilkörper 12 dieser auf die Endfläche 24 zu bewegt, bis die Gegenfläche 26 auf der Endfläche 24 aufsitzt, so dass dann der Strömungsdurchgang 16 für eine Durchströmung in einer der Exspirationsrichtung E entgegengesetzten Richtung gesperrt ist. Alternativ zu den Darstellungen der Fig. 1, 2 und 5 kann der Ventilkörper 12 mit der Gegenfläche 26 bereits in dem von einer Atemgasströmung eines Patienten unbelasteten Bezugszustand auf der Endfläche 24 aufsitzen.

Der stromabwärtige Teil des Strömungsdurchgangs 16, also der in Exspirationsströmungsrichtung E strömungsmechanisch hinter dem Ventilkörper 12 gelegene Abschnitt, ist aus zwei Teilabschnitten gebildet: Ein erster, näher am Ventilkörper 12 gelegener stromabwärtiger Abschnitt 32 des Strömungsdurchgangs 16 ist als Ringkanal konzentrisch zum Endabschnitt 22 des Atemrohrs 18 und somit konzentrisch zum stromaufwärtigen Abschnitt 20 des Strömungsdurchgangs 16 ausgebildet. Der Ringkanal des Abschnitts 32 umgibt den stromaufwärtigen Abschnitt des Strömungsdurchgangs 16 radial außen bezogen auf den Durchgangsbahnabschnitt D1, welcher der Durchgangsbahnabschnitt sowohl für den stromaufwärtigen Abschnitt 20 wie auch für den dem Ventilkörper 12 näher gelegenen stromabwärtigen Abschnitt 32 des Strömungsdurchgangs 16 ist.

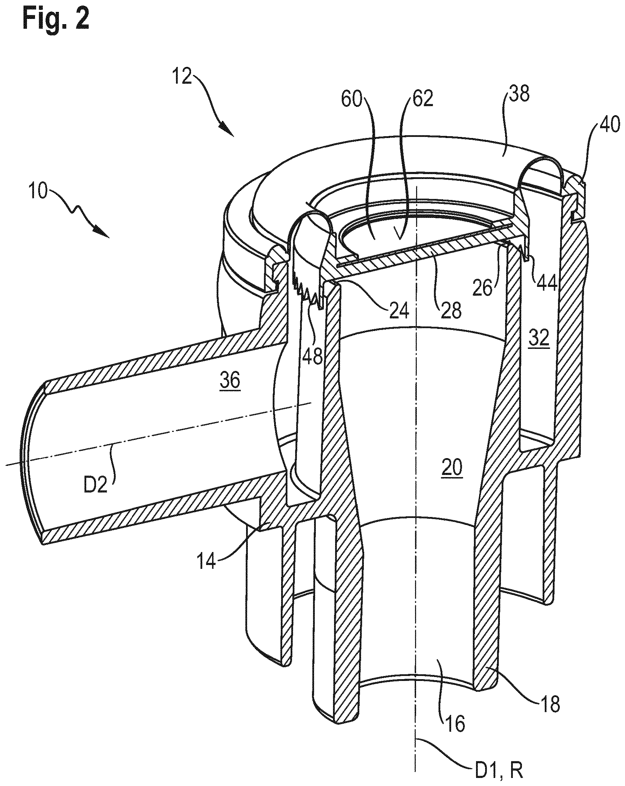

Der Strömungsdurchgang 16 umfasst eine zum distalen Ende 34 des Exspirationsventils 10 führende Stichleitung 36, welche orthogonal vom Ringkanal 32 bzw. von dem den Ringkanal bildenden, dem Ventilkörper 12 näher gelegenen Abschnitt 32 des Strömungsdurchgangs 16 ausgeht. Die Abschnitte D1 und D2 der Durchgangsbahn sind in den Fig. 1 und 2 orthogonal zueinander angeordnet und kreuzen sich bei verlängert gedachtem Abschnitt D2. Dies ist jedoch lediglich eine bevorzugte Anordnung. Abhängig von den verfügbaren Platzverhältnissen in einer das Exspirationsventil 10 aufnehmenden Beatmungsvorrichtung können die Abschnitte D1 und D2 windschief sein, d. h. einander nicht kreuzen oder/und können auch andere Winkel als den in Fig. 1 gezeigten rechten Winkel miteinander einschließen.

Der tellerartige Abschnitt 28 des Ventilkörpers 12, welcher die Gegenfläche 26 aufweist, ist im Wesentlichen eben und orthogonal zum Durchgangsbahnabschnitt D1 orientiert ausgebildet. Der Durchgangsbahnabschnitt D1 bildet außerdem eine Körperachse K, bzw. fällt mit dieser zusammen, welche den Ventilkörper 12 zentral im Wesentlichen als Rotationssymmetrieachse durchsetzt.

Der tellerartige Abschnitt 28 mit der daran ausgebildeten Gegenfläche 26 ist über eine vollständig um die Körperachse K bzw. den Strömungsdurchgangsabschnitt D1 umlaufende Membranfeder 38 mit einem radial außerhalb des Tellerabschnitts 28 ausgebildeten Befestigungsabschnitt 40 verbunden.

Der Befestigungsabschnitt 40 ist in an sich bekannter Weise formschlüssig mit einem Abschnitt des Ventilgehäuses 14 verbunden. Durch den Befestigungsabschnitt 40 ist der Ventilkörper 12 insgesamt am Ventilgehäuse 14 gehaltert. Die Membranfeder 38 zentriert den tellerartigen Abschnitt 28 relativ zum Atemrohr 18 und spannt den tellerartigen Abschnitt 28 zur Endfläche 24 hin vor bzw. setzt einem Abheben des tellerartigen Abschnitts 28 bzw. der an ihm vorgesehenen Gegenfläche 26 von der Endfläche 24 einen Widerstand entgegen. Der Widerstand ist bevorzugt umso größer, je weiter die Gegenfläche 26 längs des ersten Strömungsdurchgangsabschnitts D1 in Abheberichtung A von der Endfläche 24 entfernt ist. Die Abheberichtung A verläuft im vorliegend beschriebenen Ausführungsbeispiel längs dem ersten Abschnitt D1 der Durchgangsbahn.

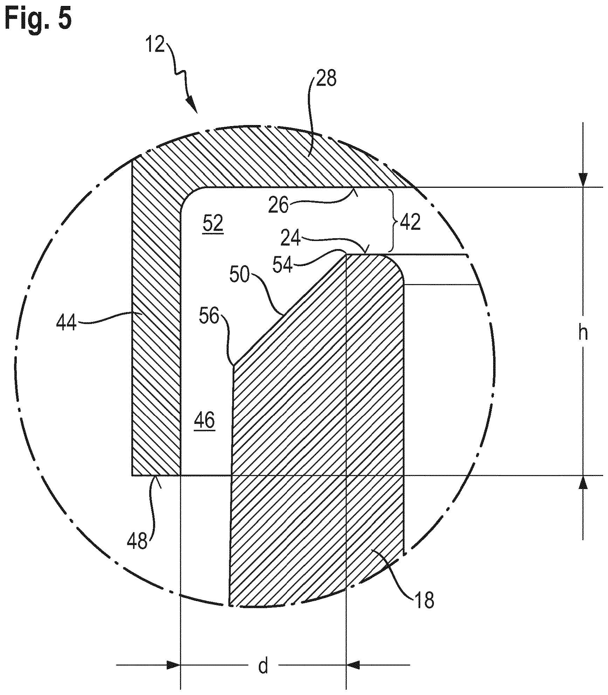

Während eines Exspirationsvorgangs eines mit dem proximalen Ende 30 des Exspirationsventils 10 verbundenen Patienten wird der Druck im stromaufwärtigen Abschnitt 20 des Strömungsdurchgangs 16 erhöht, während auf der vom Atemrohr 18 abgewandten Seite des Ventilkörpers 12 dauerhaft Umgebungsdruck herrscht. Durch die Druckerhöhung auf der angeströmten Seite des tellerartigen Abschnitts 28 wirkt auf den tellerartigen Abschnitt 28 eine in Abheberichtung A wirkende Drucckraft, welche mit zunehmendem Druckunterschied die elastische Kraft der Membranfeder 38 überwindet, so dass der tellerartige Abschnitt 28 und mit ihm die Gegenfläche 26 längs der Abheberichtung A von der Endfläche 24 weg verlagert werden. Es wird dadurch ein zwischen der Endfläche 24 und der Gegenfläche 26 gebildeter oder bestehender Ringspalt 42 (siehe Fig. 5) vergrößert. Der Strömungswiderstand in Exspirationsrichtung E zwischen der Endfläche 24 und der Gegenfläche 26 nimmt dadurch ab, so dass eine Exspirationsströmung vom proximalen Ende 30 zum distalen Ende 34 des Exspirationsventils 10 nahezu ungestört möglich ist.

Die in Exspirationsströmungsrichtung E verlaufende Exspirationsströmung wird am tellerartigen Abschnitt 28 des Ventilkörpers 12 zwangsweise umgelenkt, so dass sie bezogen auf den Durchgangsbahnabschnitt D1 in radialer Richtung vom Durchgangsbahnabschnitt D1 weg durch den Ringspalt 42 strömt.

Zur Stabilisierung dieser Exspirationsströmung im unmittelbar stromabwärts des Ringspalts 42 zwischen Endfläche 24 und Gegenfläche 26 gelegenen Abschnitt des Strömungsdurchgangs 16 weist der Ventilkörper 12 eine Schürze 44 auf, welche ein ausschließlich radiales Abströmen der Exspirationsströmung nach dem Durchgang durch den Ringspalt 42 verhindert und die Exspirationsströmung erneut umlenkt, diesmal in eine Strömungsrichtung mit einer der Exspirationsströmungsrichtung unmittelbar stromaufwärts des Ventilkörpers 12 entgegengesetzten Richtungskomponente.

Die Schürze 44 läuft in Umfangsrichtung um die Körperachse K des Ventilkörpers 12 vollständig um und umgibt sowohl die Gegenfläche 26 als auch die Endfläche 24. Die Schürze 44 steht hierfür entgegen der Abheberichtung A von dem die Gegenfläche 26 aufweisenden tellerartigen Abstand 28 vor, und zwar so weit, dass sie nicht nur entgegen der Abheberichtung A die Endfläche 24 überragt, sondern einen die Endfläche 24 aufweisenden axialen Endabschnitt des Atemrohrs 18 radial außen vollständig umgibt. Zwischen dem Atemrohr 18 und der Schürze 44 ist ein Ringspaltraum 46 gebildet (siehe Fig. 5), welcher ein Abströmen der Exspirationsströmung selbst bei nur geringfügig von der Endfläche 24 abgehobener Gegenfläche 26 ermöglicht.

Durch die den Ringspalt 42 wenigstens in der Bezugsstellung des Exspirationsventils 10 vollständig umgebende Schürze wird eine Stabilisierung der Expansionsströmung im Bereich des Ventildurchgangs erreicht, der im Vergleich zu einem gleichartigen Ventilkörper 12 ohne Schürze, wie er im Stand der Technik bisher verwendet wurde, erheblich weniger zu Wirbelbildung und Strömungsabrissen neigt und dadurch eine Exspirationsströmung unter erheblich geringerer Geräuschbildung ermöglicht. Versuche haben gezeigt, dass ein erfindungsgemäßes Exspirationsventil vor allem in einem Volumenströmungsbereich von etwa 15 Liter pro Minute verglichen mit Exspirationsventilen des Standes der Technik eine erheblich verminderte Geräuschentwicklung zeigt.

Die Schürze 44 kann sich entgegen der Abheberichtung A so weit vom tellerartigen Abschnitt 28 und der an ihm ausgebildeten Gegenfläche 26 weg erstrecken, dass ein Ringspaltraum 46 zwischen der Schürze 44 und der Außenseite des Atemrohrs 18 bis zum Überschreiten eines vorbestimmten Abhebebetrags der Gegenfläche 26 von der Endfläche 24 bestehen bleibt.

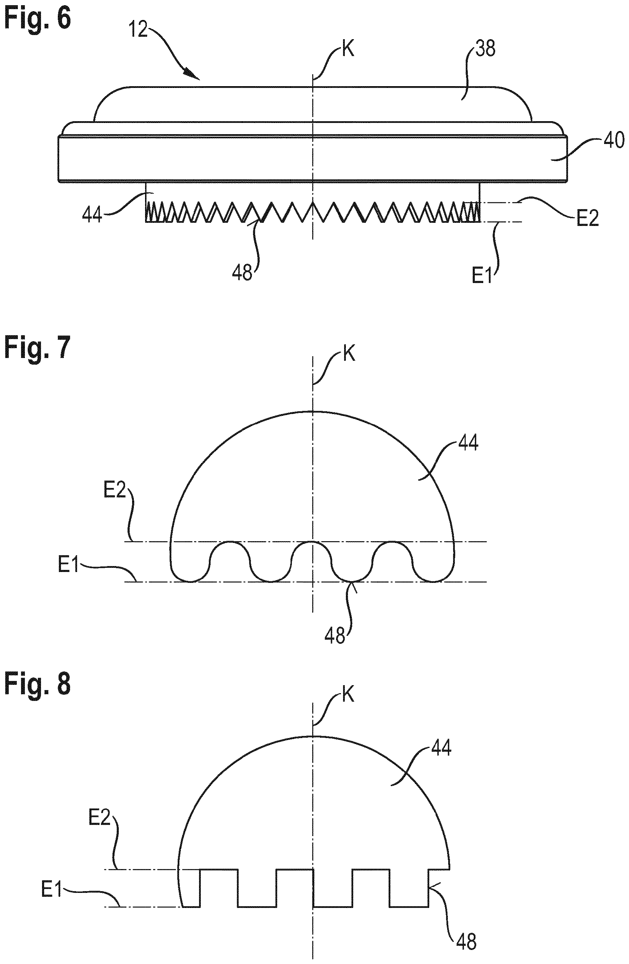

Der freie Rand 48 der Schürze 44 kann, wie in Fig. 1 gezeigt ist, ein glatter Rand sein, welcher längs einer Kreisbahn um die Körperachse K des Ventilkörpers 12 umläuft. Der freie Rand 48 der Schürze 44 kann jedoch auch, wie in den Fig. 2 und 6 gezeigt ist, eine Wellenform aufweisen, beispielsweise eine Dreiecks-Wellengestalt mit in Umfangsrichtung aufeinanderfolgenden gleich großen gleichschenkligen Dreiecken. Alternativ kann, wie in Fig. 7 dargestellt ist, der Rand 48 der Schürze 44 teilkreisförmig oder sinusförmig ausgestaltet sein oder kann, wie in Fig. 8 gezeigt ist, als Rechteck-Wellenform ausgestaltet sein. Wie in Fig. 5 gezeigt ist, kann das Atemrohr 18 radial außerhalb der Endfläche 24 mit einer Fase 50 versehen sein. Die Fase 50 läuft vorzugsweise vollständig um die Rohrachse R des Atemrohrs 18 um und bildet im Zusammenwirken mit dem tellerartigen Abschnitt 28 und der davon entgegen der Abheberichtung A abstehenden Schürze 44 einen nach radial außen wirkenden Expansionsraum 52 (siehe Fig. 5), in welchen hinein die den Ringspalt 42 zwischen Endfläche 24 und Gegenfläche 26 radial durchströmende Exspirationsströmung entspannen kann.

Wie in Fig. 5 gezeigt ist, ist bevorzugt die Auskraglänge h, mit welcher die Schürze 44 entgegen der Abheberichtung A vom tellerartigen Abschnitt 28 des Ventilkörpers 12 absteht, größer als der radiale Abstand d zwischen dem der Endfläche 24 näher gelegenen Ende 54 der Fase 50 und einer nach radial innen weisenden Wandung der Schürze 44.

Wie Fig. 5 weiter zeigt, erstreckt sich die Schürze 44 entgegen der Abheberichtung A nicht nur über die Endfläche 24, sondern auch über das von der Endfläche 24 ferner liegende Ende 56 der Fase 50 hinaus.

Bei einer wellenförmigen Ausbildung des Randes 48 der Schürze 44 ist die Auskraglänge h bis zu einem von der Gegenfläche 26 fernstliegenden Extrempunkt, also unter Vernachlässigung der Wellentäler, zu bestimmen.

Wie an den Fig. 6, 7 und 8 zu erkennen ist, liegen die von der Gegenfläche 26 am weitest entfernt gelegenen Extrempunkte der Wellenberge auf einer ersten zur Körperachse K des Ventilkörpers 12 orthogonalen Ebene E1 und liegen die der Gegenfläche 26 nächstgelegenen Extrempunkte der Wellentäler auf einer zweiten zur ersten parallelen Ebene E2. Die Ebenen E1 und E2 verlaufen jeweils orthogonal zur Körperachse K des Ventilkörpers 12.

Auf seiner vom Atemrohr 18 abgewandten Seite weist der tellerartige Abschnitt 28 des Ventilkörpers 12 bevorzugt eine Verstärkungsscheibe 60 auf, welche die Gestalt des tellerartigen Abschnitts 28 stabilisiert. Der übrige Ventilkörper 12 mit Ausnahme der Verstärkungsscheibe 60 ist vorzugsweise einstückig aus einem weichelastischen Elastomer gebildet, beispielsweise aus Silikon, Kautschuk oder Naturkautschuk.

Die Verstärkungsscheibe 60 bildet nicht nur eine Formstabilisierung des tellerartigen Abschnitts 28, sondern bildet mit ihrer stabilen und harten nach außen freiliegenden Oberfläche 62 (siehe Fig. 2) eine Angriffsfläche für einen Aktuator 63 zur erzwungenen Verlagerung des tellerartigen Abschnitts 28 - und mit ihm der Gegenfläche 26 - zur Endfläche 24 hin. Der Übersichtlichkeit halber ist in Fig. 1 lediglich ein halber Aktuatorstößel mit 64 strichliniert dargestellt und angedeutet.

Der Aktuatorstößel 64 kann durch Absenken zur Verstärkungsscheibe 60 mit dieser in Anlageeingriff gebracht werden. Nach Herstellung des Anlageeingriffs kann durch weiteres Absenken des Aktuatorstößels 64 der vollständige tellerartige Abschnitt 28 samt Verstärkungsscheibe zum Endabschnitt 22 des Atemrohrs 18 hin bewegt werden. Durch Zurückziehen des Stößels 64 in Abheberichtung A kann dieser von der Verstärkungsscheibe 60 abgehoben werden. Der Stößel 64 kann elektromagnetisch zur Bewegung angetrieben sein. Ebenfalls kann er durch einen elektromotorischen Antrieb mittels eines Getriebes zur Bewegung längs der Körperachse A angetrieben sein.

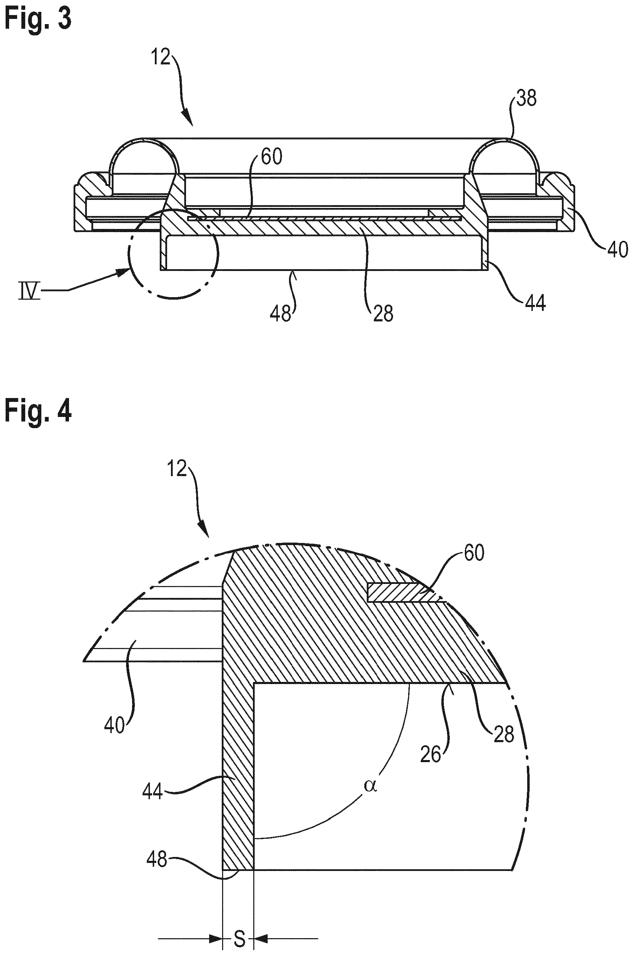

In Fig. 4 ist ein Detailschnitt des Ventilkörpers 12 von Fig. 3 vergrößert dargestellt.

Die Schürze 44 schließt bevorzugt in der die Körperachse K des Ventilkörpers 12 einschließenden Schnittebene einen Winkel α mit der Ebene der vorzugsweise ebenen Gegenfläche 26 ein, welcher 90° oder geringfügig größer als 90° ist. Bevorzugt liegt der Winkel α in einem Bereich von 90° bis 95°, besonders bevorzugt bis 92,5°.

Die radiale Dicke s der Schürze 44 ist bevorzugt längs ihrer Auskragstrecke h mit Ausnahme einer unvermeidlichen Übergangskrümmung am Übergang zum tellerartigen Abschnitt 28 im Wesentlichen konstant. Sie ist bevorzugt um nicht mehr als 10% größer oder kleiner als die radiale Erstreckung des Ringspaltraums 46 zwischen der nach radial außen weisenden Fläche des Atemrohrs 18 und der nach radial innen weisenden Wandungsfläche der Schürze 44.

Mit dem vorliegend beschriebenen Exspirationsventil ist es möglich, ohne Erhöhung des Strömungswiderstands die Geräuschentwicklung bei der Durchströmung während eines Exspirationsvorgangs erheblich zu vermindern. Ebenso kann mit dem Exspirationsventil, wie es oben vorgestellt wurde, eine Durchströmung des Ventilgehäuses 14 in einer der Exspirationsströmungsrichtung E entgegengesetzten Strömungsrichtung sicher verhindert werden.

## Patentansprüche

1. Exspirationsventil (10) für eine Beatmungsvorrichtung zur wenigstens teilweise apparativ unterstützten Beatmung eines Patienten, umfassend ein Ventilgehäuse (14) mit einem sich längs einer eine lokale axiale, radiale und Umfangsrichtung definierenden Durchgangsbahn (D1, D2) erstreckenden Strömungsdurchgang (16), längs welchem das Ventilgehäuse (14) mit Atemluft durchströmbar ist, wobei das Ventilgehäuse (14) eine gehäusefeste Ventil-Teilformation mit einer geschlossen um die Durchgangsbahn (D1) umlaufenden Endfläche (24) aufweist, wobei eine zur Endfläche (24) hinweisende Gegenfläche (26) eines relativ zum Ventilgehäuse (14) beweglichen Ventilkörpers (12) durch eine Vorspanneinrichtung (38) derart vorgespannt ist, dass die Gegenfläche (26) durch Anströmen mit Atemgas in einer Exspirationsströmungsrichtung (E) gegen eine Vorspannkraft der Vorspanneinrichtung (38) unter Vergrößerung eines zwischen der Endfläche (24) und der Gegenfläche (26) erzeugbaren oder vorhandenen Ringspalts (42) in einer Abheberichtung (A) von der Endfläche (24) entfernbar ist, so dass der Strömungsdurchgang (16) in der Exspirationsströmungsrichtung (E) durchströmbar ist und eine Durchströmung des Strömungsdurchgangs (16) in eine der Exspirationsströmungsrichtung (E) entgegengesetzte Strömungsrichtung durch Anlage der Gegenfläche (26) des Ventilkörpers (12) an der Endfläche (24) sperrbar ist, wobei der Ventilkörper (12) eine Schürze (44) aufweist, welche, - bei Betrachtung des Exspirationsventils (10) in einem durch bestimmungsgemäße Atemströmung unbelasteten Bezugszustand - die Gegenfläche (26) und die Endfläche (24) umgebend, sich in einer Umfangsrichtung erstreckt und welche im Bezugszustand entgegen der Abheberichtung (A) in Richtung von der Gegenfläche (26) weg über die Endfläche (24) hinaus absteht, wobei radial zwischen der Schürze (44) und einem die Endfläche (24) aufweisenden Endabschnitt (22) der Ventil-Teilformation ein Ringspaltraum (46) vorgesehen ist,
**dadurch gekennzeichnet, dass** ein parallel zur Abheberichtung (A) zu messender Abstand (h) eines von der Gegenfläche (26) fernen Schürzenrandes (48) von der Gegenfläche (26) wenigstens umfangsabschnittsweise abhängig von der jeweiligen Position in Umfangsrichtung unterschiedlich groß ist.

2. Exspirationsventil (10) nach Anspruch 1,
**dadurch gekennzeichnet, dass** der von der Gegenfläche (26) ferne Schürzenrand (48) eine in Umfangsrichtung verlaufende Wellenform aufweist.

3. Exspirationsventil (10) nach Anspruch 2,
**dadurch gekennzeichnet, dass** die Wellenform eine Dreiecks- oder/und Rechtecks- oder/und Teilkreis-Wellenform oder/und eine sinusartige Wellenform aufweist.

4. Exspirationsventil (10) nach Anspruch 2 oder 3,
**dadurch gekennzeichnet, dass** wenigstens ein Teil, vorzugsweise alle, der entgegen der Abheberichtung (A) von der Gegenfläche (26) fernstgelegenen Extrempunkte der Wellenberge oder/und dass wenigstens ein Teil, vorzugsweise alle, der entgegen der Abheberichtung (A) der Gegenfläche nächstgelegenen Extrempunkte der Wellentäler auf einer Ebene (E1, E2) gelegen sind, insbesondere auf einer Ebene (E1, E2), welche zum Verlauf der Durchgangsbahn (D1, D2) am Durchstoßpunkt der Ebene oder/und zur Abheberichtung (A) orthogonal ist.

5. Exspirationsventil (10) nach einem der vorhergehenden Ansprüche,
**dadurch gekennzeichnet, dass** die Gegenfläche (26) oder/und die Endfläche (24) in einer Ebene gelegen ist bzw. sind, vorzugsweise eben ist bzw. sind.

6. Exspirationsventil (10) nach einem der vorhergehenden Ansprüche,
**dadurch gekennzeichnet, dass** die Endfläche (24) an einem Längsende (22) eines rohrförmigen Abschnitts (18) des Strömungsdurchgangs (16) als dem Endabschnitt (22) der Ventil-Teilformation gelegen ist, wobei die Schürze (44) im Bezugszustand einen sich längs der Durchgangsbahn (D1, D2) und in Umfangsrichtung um diesen herum erstreckenden Endbereich (22) des rohrförmigen Abschnitts (18) umgibt.

7. Exspirationsventil (10) nach Anspruch 6,
**dadurch gekennzeichnet, dass** ein radial äußerer Bereich des Längsendes (22) des rohrförmigen Abschnitts (18) angefast ist, wobei bevorzugt die Schürze (44) im Bezugszustand entgegen der Abheberichtung (A) über das axial weiter von der Endfläche (24) entfernt gelegene Fasenende (56) hinausreicht.

8. Exspirationsventil (10) nach Anspruch 7,
**dadurch gekennzeichnet, dass** sich im Bezugszustand der radiale Abstand des näher an der Endfläche (24) gelegenen Fasenendes (54) von einer nach radial innen weisenden Wandung der Schürze (44) und die Überlappungstiefe von Schürze (44) und rohrförmigem Abschnitt (18) parallel zur Abheberichtung (A) um nicht mehr als 20 %, vorzugsweise um nicht mehr als 10 % unterscheiden, besonders bevorzugt gleich sind.

9. Exspirationsventil (10) nach einem der vorhergehenden Ansprüche,
**dadurch gekennzeichnet, dass** sich im Bezugszustand die radiale Abmessung des Ringspaltraums (46) und die radiale Dicke (s) der Schürze (44) in einem den von der Gegenfläche (26) fernliegenden Schürzenrand (48) enthaltenden Endbereich der Schürze (44) um nicht mehr als 20 %, vorzugsweise um nicht mehr als 10 % unterscheiden, besonders bevorzugt gleich sind.

10. Exspirationsventil (10) nach einem der vorhergehenden Ansprüche,
**dadurch gekennzeichnet, dass** ein Teil (20) des Strömungsdurchgangs (16) durch ein Atemrohr (18) und einen das Atemrohr (18), vorzugsweise koaxial, umgebenden Ringkanal (32) gebildet ist, wobei der Ringspalt (42) zwischen Endfläche (24) und Gegenfläche (26) strömungsmechanisch in Exspirationsströmungsrichtung (E) zwischen dem Atemrohr (18) und dem Ringkanal (32) gebildet ist.

11. Exspirationsventil (10) nach einem der vorhergehenden Ansprüche,
**dadurch gekennzeichnet, dass** der Ventilkörper (12) einen die Gegenfläche (26) aufweisenden, im Wesentlichen ebenen Tellerabschnitt (28) aufweist, welcher bevorzugt mittels einer Membranfeder (38) als der Vorspanneinrichtung (38) mit einem den Tellerabschnitt (38) radial außen umgebenden Befestigungsabschnitt (40) verbindet.

12. Exspirationsventil (10) nach Anspruch 11,
**dadurch gekennzeichnet, dass** der Tellerabschnitt (28) durch ein Verstärkungsbauteil (60), wie etwa Metall- oder/und Keramikscheibe, verstärkt ist, wobei bevorzugt das Verstärkungsbauteil (60) auf der von der Endfläche (26) weg weisenden Seite des Tellerabschnitts (28) wenigstens abschnittsweise freil iegt.

13. Exspirationsventil (10) nach einem der vorhergehenden Ansprüche,
**dadurch gekennzeichnet, dass** die Vorspanneinrichtung (38) die Gegenfläche (26) in einer zur Abheberichtung orthogonalen Ebene in eine vorbestimmte Ruhestellung vorspannt, insbesondere relativ zur Durchgangsbahn (D1, D2) im Bereich der Gegenfläche (26) zentriert, oder/und während einer Abhebe- und Rückstellbewegung in beziehungsweise entgegen der Abheberichtung (A) führt.

14. Exspirationsventil (10) nach einem der vorhergehenden Ansprüche,
**dadurch gekennzeichnet, dass** mit dem Ventilgehäuse (14) ein Aktuator (63) verbunden ist, von welchem ein Stellglied (64) wenigstens zur Verlagerung der Gegenfläche (26) entgegen der Abheberichtung (A) mit dem Ventilkörper (12) zusammenwirkt.

15. Exspirationsventil (10) nach Anspruch 14,
**dadurch gekennzeichnet, dass** das Stellglied (64) des Aktuators (63) mit dem Ventilkörper zur Verlagerung der Gegenfläche sowohl in als auch entgegen der Abheberichtung gekoppelt oder koppelbar ist.

16. Beatmungsvorrichtung zur wenigstens teilweisen apparativ unterstützten Beatmung eines Patienten mit einer Atemgas-Förderpumpe, mit einem Exspirationsventil (10) nach einem der vorhergehenden Ansprüche und mit einem Inspirationsventil.

17. Ventilkörper (12) für ein Exspirationsventil (10), insbesondere für ein Exspirationsventil (10) der Ansprüche 1 bis 15, umfassend eine zur Anlage an einer Ventilsitzfläche (24) ausgebildete Anlagefläche (26), welche längs einer Bewegungsachse (K) in und entgegen einer Abheberichtung (A) bewegbar ist, wobei die Anlagefläche (26) mit der Bewegungsachse (K) einen, vorzugsweise rechten, Winkel einschließt, wobei der Ventilkörper (12) eine Schürze (44) aufweist, welche die Anlagefläche (26) bezüglich der Bewegungsachse (K) radial außen umgibt und welche ausgehend von einem die Anlagefläche aufweisenden Ventilkörperabschnitt (28) bezüglich der Bewegungsachse (K) axial von dem Ventilkörperabschnitt (28) auskragt und dabei die Anlagefläche (26) axial überragt,
**dadurch gekennzeichnet, dass** ein parallel zur Abheberichtung (A) zu messender Abstand (h) eines von der Anlagefläche (26) fernen Schürzenrandes (48) von der Anlagefläche (26) wenigstens umfangsabschnittsweise abhängig von der jeweiligen Position in Umfangsrichtung unterschiedlich groß ist.

## Claims

1. An exhalation valve (10) for a ventilation apparatus for at least partly mechanically assisted ventilation of a patient, encompassing a valve housing (14) having a flow passage (16) which extends along a passage path (D1, D2) that defines a local axial, radial, and circumferential direction, and along which respiratory air can flow through the valve housing (14); the valve housing (14) comprising a housing-mounted valve sub-configuration having an end surface (24) which continuously encircles the passage path (D1); a counterpart surface (26), facing toward the end surface (24), of a valve body (12) movable relative to the valve housing (14) being preloaded by a preloading device (38) in such a way that as a result of impingement of a flow of respiratory gas in an exhalation flow direction (E), the counterpart surface (26) is movable away from the end surface (24) in a lifting direction (A) against a preload force of the preloading device (38), accompanied by enlargement of an annular gap (42) generatable or present between the end surface (24) and the counterpart surface (26), so that the flow passage (16) is flowthrough-capable in the exhalation flow direction (E) and so that a flow through the flow passage (16) in a flow direction opposite to the exhalation flow direction (E) is blockable by abutment of the counterpart surface (26) of the valve body (12) against the end surface (24),
wherein the valve body (12) comprises a skirt (44) which, when considering the exhalation valve (10) in a reference state not stressed by a respiratory flow as intended, extends in a circumferential direction surrounding the counterpart surface (26) and the end surface (24), and which in the reference state projects axially beyond the end surface (24), oppositely to the lifting direction (A), in a direction away from the counterpart surface (26), an annular gap space (46) being provided radially between the skirt (44) and an end portion (22), facing toward the end surface (24), of the valve sub-configuration, **characterized in that** the magnitude of a spacing (h), to be measured parallel to the lifting direction (A), between a skirt rim (48) remote from the counterpart surface (26) and the counterpart surface (26) is different at least circumferentially locally depending on the respective position in a circumferential direction.

2. The exhalation valve (10) according to Claim 1,
**characterized in that** the skirt rim (48) remote from the counterpart surface (26) exhibits a wave shape proceeding in a circumferential direction.

3. The exhalation valve (10) according to Claim 2,
**characterized in that** the wave shape has a triangular and/or rectangular and/or partial-circle wave shape and/or a sinusoidal wave shape.

4. The exhalation valve (10) according to Claim 2 or 3,
**characterized in that** at least some, preferably all, of the extreme points of the wave crests located farthest from the counterpart surface (26) oppositely to the lifting direction (A), and/or at least some, preferably all, of the extreme points of the wave troughs located closest to the counterpart surface oppositely to the lifting direction (A), are located on one plane (E1, E2), in particular on a plane (E1, E2) that is orthogonal to the course of the passage path (D1, D2) at the penetration point of the plane and/or orthogonal to the lifting direction (A).

5. The exhalation valve (10) according to one of the preceding claims,
**characterized in that** the counterpart surface (26) and/or the end surface (24) is/are located in one plane, preferably is/are flat.

6. The exhalation valve (10) according to one of the preceding claims,
**characterized in that** the end surface (24) is located at a longitudinal end (22) of a tubular portion (18) of the flow passage (16) constituting the end portion (22) of the valve sub-configuration; the skirt (44) surrounding, in the reference state, an end region (22), extending along the passage path (D1, D2) and in a circumferential direction around it, of the tubular portion (18).

7. The exhalation valve (10) according to Claim 6,
**characterized in that** a radially outer region of the longitudinal end (22) of the tubular portion (18) is beveled, the skirt (44) preferably extending, in the reference state, oppositely to the lifting direction (A) beyond the bevel end (56) located axially farther from the end surface (24).

8. The exhalation valve (10) according to Claim 7,
**characterized in that** in the reference state, the radial spacing between the bevel end (54) located closer to the end surface (24) and a radially inward-facing wall of the skirt (44), and the overlap depth of the skirt (44) and the tubular portion (18) parallel to the lifting direction (A), differ by no more than 20%, preferably by no more than 10%, particularly preferably are identical.

9. The exhalation valve (10) according to one of the preceding claims,
**characterized in that** in the reference state, the radial dimension of the annular gap space (46) and the radial thickness (s) of the skirt (44) differ, in an end region of the skirt (44) containing the skirt rim (48) located remotely from the counterpart surface (26), by no more than 20%, preferably by no more than 10%, particularly preferably are identical.

10. The exhalation valve (10) according to one of the preceding claims,
**characterized in that** a part (20) of the flow passage (16) is constituted by a respiration tube (18) and by an annular channel (32) surrounding the respiration tube (18), preferably coaxially, the annular gap (42) between the end surface (24) and counterpart surface (26) being constituted in terms of flow mechanics between the respiration tube (18) and the annular channel (32) in the exhalation flow direction (E).

11. The exhalation valve (10) according to one of the preceding claims,
**characterized in that** the valve body (12) comprises a substantially flat plate portion (28) which comprises the counterpart surface (26) and which connects, preferably by means of a diaphragm spring (38) constituting the preloading device (38), to a fastening portion (40) radially externally surrounding the plate portion (28).

12. The exhalation valve (10) according to Claim 11,
**characterized in that** the plate portion (28) is reinforced by a reinforcing component (60), for example a metal disk and/or a ceramic disk, the reinforcing component (60) preferably being exposed at least in portions on that side of the plate portion (28) which faces away from the end surface (26).

13. The exhalation valve (10) according to one of the preceding claims,
**characterized in that** the preloading device (38) preloads the counterpart surface (26), in a plane orthogonal to the lifting direction, into a predetermined idle position, in particular centers it relative to the passage path (D1, D2) in the region of the counterpart surface (26), and/or guides it during a lifting and return motion respectively in and oppositely to the lifting direction (A).

14. The exhalation valve (10) according to one of the preceding claims,
**characterized in that** an actuator (63), a positioning member (64) of which interacts with the valve body (12) at least in order to displace the counterpart surface (26) oppositely to the lifting direction (A), is connected to the valve housing (14).

15. The exhalation valve (10) according to Claim 14,
**characterized in that** the positioning member (64) of the actuator (63) is coupled or couplable to the valve body for displacement of the counterpart surface both in and oppositely to the lifting direction.

16. A ventilation apparatus for at least partly mechanically assisted ventilation of a patient, having a respiratory gas conveying pump, having an exhalation valve (10) according to one of the preceding claims, and having an inhalation valve.

17. A valve body (12) for an exhalation valve (10), in particular for an exhalation valve (10) of Claims 1 to 16, encompassing an abutment surface (26) that is embodied for abutment against a valve seat surface (24) and is movable along a motion axis (K) in and oppositely to a lifting direction (A); the abutment surface (26) enclosing a, preferably right, angle with the motion axis (K); the valve body (12) comprising a skirt (44) which surrounds the abutment surface (26) radially externally with reference to the motion axis (K) and which, proceeding from a valve body portion (28) comprising the abutment surface, protrudes from the valve body portion (28) axially with reference to the motion axis (K) and in that context projects axially beyond the abutment surface (26), **characterized in that** the magnitude of a spacing (h), to be measured parallel to the lifting direction (A), between a skirt rim (48) remote from the counterpart surface (26) and the counterpart surface (26) is different at least circumferentially locally depending on the respective position in a circumferential direction.

## Revendications

1. Soupape d'expiration (10) pour un dispositif respiratoire pour la respiration au moins partiellement assistée par un appareil d'un patient, comprenant un boîtier de soupape (14) avec un passage d'écoulement (16) s'étendant le long d'un trajet de passage (D1, D2) définissant une direction locale axiale, radiale et circonférentielle, le long duquel de l'air respiratoire peut s'écouler à travers le boîtier de soupape (14), le boîtier de soupape (14) comprenant une formation partielle de soupape fixée au boîtier avec une surface d'extrémité (24) s'étendant de manière fermée autour du passage d'écoulement (D1), dans lequel une contre-surface (26) d'un corps de soupape (12) mobile par rapport au boîtier de soupape (14) et orientée vers la surface d'extrémité (24) est sollicitée par un dispositif de sollicitation (38) de telle manière que que la contre-surface (26) peut être retirée de la surface d'extrémité (24) en faisant circuler contre elle du gaz respiratoire dans une direction d'écoulement expiratoire (E) à l'encontre d'une force de sollicitation du dispositif de sollicitation (38) tout en élargissant un espace annulaire (42), qui peut être produit ou existe entre la surface d'extrémité (24) et la contre-surface (26), dans une direction de soulèvement (A), de sorte que le passage d'écoulement (16) peut être traversé dans la direction d'écoulement expiratoire (E) et qu'un écoulement à travers le passage d'écoulement (16) dans une direction d'écoulement opposée à la direction d'écoulement expiratoire (E) peut être bloqué par la mise en butée de la contre-surface (26) du corps de soupape (12) contre la surface d'extrémité (24), le corps de soupape (12) comprenant une jupe (44), laquelle, - lorsque la soupape d'expiration (10) est vue dans un état de référence non chargé par le flux respiratoire prévu - entourant la contre-surface (26) et la surface d'extrémité (24), s'étend dans une direction circonférentielle et qui, dans l'état de référence, fait saillie dans la direction s'éloignant de la contre-surface (26) au-delà de la surface d'extrémité (24) dans la direction opposée à la direction de soulèvement (A), dans lequel un espace annulaire (46) est prévu radialement entre la jupe (44) et une partie d'extrémité (22) de la partie de soupape ayant la surface d'extrémité (24),
**caractérisé en ce qu'**une distance (h), à mesurer parallèlement à la direction de soulèvement (A), d'un bord de jupe éloigné de la contre-surface (26) par rapport à la contre-surface (26) varie, au moins dans des sections circonférentielles, en fonction de la position respective dans la direction circonférentielle.

2. Soupape d'expiration (10) selon la revendication 1,
**caractérisée en ce que** le bord de jupe (48) éloigné de la contre-surface (26) a une forme ondulée s'étendant dans la direction circonférentielle.

3. Soupape d'expiration (10) selon la revendication 2,
**caractérisée en ce que** la forme d'onde a une forme triangulaire ou/et rectangulaire ou/et de cercle partiel ou/et sinusoïdale.

4. Soupape d'expiration (10) selon la revendication 2 ou 3,
**caractérisée en ce qu'**au moins une partie, de préférence la totalité, des points extrêmes des crêtes d'onde le plus éloignés de la contre-surface (26) à l'opposé de la direction de soulèvement (A) ou/et qu'au moins une partie, de préférence la totalité, des points extrêmes des creux d'onde le plus près de la contre-surface à l'opposé de la direction de soulèvement (A) sont situés sur un plan (E1, E2), en particulier sur un plan (E1, E2) qui est orthogonal au trajet de passage (D1, D2) au point d'intersection du plan et/ou de la direction de soulèvement (A).

5. Soupape d'expiration (10) selon l'une des revendications précédentes,
**caractérisée en ce que** la contre-surface (26) ou/et la surface d'extrémité (24) est/sont située(s) dans un plan, de préférence est/sont plate(s).

6. Soupape d'expiration (10) selon l'une des revendications précédentes,
**caractérisée en ce que** la surface d'extrémité (24) est située à une extrémité longitudinale (22) d'une partie tubulaire (18) du passage d'écoulement (16) en tant que partie d'extrémité (22) de la sous-formation de soupape, dans laquelle la jupe (44) dans l'état de référence entoure une partie d'extrémité (22) de la partie tubulaire (18) s'étendant le long du trajet de passage (D1, D2) et circonférentiellement autour de celui-ci.

7. Soupape d'expiration (10) selon la revendication 6,
**caractérisée en ce qu'**une région radialement extérieure de l'extrémité longitudinale (22) de la section tubulaire (18) est chanfreinée, dans laquelle de préférence la jupe (44) s'étend, à l'état de référence, à l'opposé de la direction de soulèvement (A) au-delà de l'extrémité de chanfrein (56) située axialement plus loin de la surface d'extrémité (24).

8. Soupape d'expiration (10) selon la revendication 7,
**caractérisé en ce que,** dans l'état de référence, la distance radiale de l'extrémité de chanfrein (54) située plus près de la surface d'extrémité (24) par rapport à une paroi de la jupe (44) pointant radialement vers l'intérieur et la profondeur de recouvrement de la jupe (44) et de la section tubulaire (18) parallèlement à la direction de soulèvement (A) ne diffèrent pas de plus de 20 %, de préférence pas de plus de 10 %, et sont de manière particulièrement préférée identiques.

9. Soupape d'expiration (10) selon l'une des revendications précédentes,
**caractérisé en ce que,** dans l'état de référence, la dimension radiale de l'espace annulaire (46) et l'épaisseur radiale (s) de la jupe (44) dans une région d'extrémité de la jupe (44) contenant le bord de la jupe (48) éloigné de la contre-surface (26) ne diffèrent pas de plus de 20 %, de préférence pas de plus de 10 %, et sont de manière particulièrement préférée identiques.

10. Soupape d'expiration (10) selon l'une des revendications précédentes,
**caractérisée en ce qu'**une partie (20) du passage d'écoulement (16) est formée par un tube de respiration (18) et un canal annulaire (32) entourant le tube de respiration (18), de préférence coaxialement, dans laquelle l'espace annulaire (42) entre la surface d'extrémité (24) et la contre-surface (26) est formé de manière fluide dans la direction d'écoulement d'expiration (E) entre le tube de respiration (18) et le canal annulaire (32).

11. Soupape d'expiration (10) selon l'une des revendications précédentes,
**caractérisée en ce que** le corps de soupape (12) comprend une partie de disque sensiblement plane (28) comprenant la contre-surface (26), de préférence reliée au moyen d'un ressort à diaphragme (38) comme moyen de sollicitation (38) à une partie de fixation (40) entourant radialement vers l'extérieur la partie de disque (38).

12. Soupape d'expiration (10) selon la revendication 11,
**caractérisée en ce que** la partie de disque (28) est renforcée par un composant de renforcement (60), tel qu'un disque métallique ou/et céramique, dans laquelle de préférence le composant de renforcement (60) est exposé au moins par sections sur le côté de la partie de disque (28) opposé à la face d'extrémité (26).

13. Soupape d'expiration (10) selon l'une des revendications précédentes,
**caractérisée en ce que** le dispositif de sollicitation (38) précontraint la contre-surface (26) dans une position de repos prédéterminée dans un plan orthogonal à la direction de soulèvement, en particulier centrée par rapport au passage (D1, D2) dans la région de la contre-surface (26), et/ou la guide dans ou contre la direction de soulèvement (A) pendant un mouvement de soulèvement et de retour.

14. Soupape d'expiration (10) selon l'une des revendications précédentes,
**caractérisée en ce qu'**un actionneur (63) est relié au boîtier de soupape (14), dont un élément d'actionneur (64) coopère avec le corps de soupape (12) au moins pour déplacer la contre-surface (26) à l'opposé de la direction de soulèvement (A).

15. Soupape d'expiration (10) selon la revendication 14,
**caractérisée en ce que** l'élément d'actionnement (64) de l'actionneur (63) est couplé ou peut être couplé au corps de soupape pour déplacer la contre-surface à la fois dans et contre la direction de soulèvement.

16. Dispositif respiratoire pour la respiration au moins partiellement assistée par un appareil d'un patient, comprenant une pompe d'administration de gaz respiratoire, comprenant une soupape d'expiration (10) selon l'une des revendications précédentes et comprenant une soupape d'inspiration.

17. Corps de soupape (12) pour une soupape d'expiration (10), en particulier pour une soupape d'expiration (10) selon les revendications 1 à 15, comprenant une surface de butée (26) conçue pour s'appuyer contre une surface de siège de soupape (24), laquelle surface de butée (26) est mobile le long d'un axe de mouvement (K) dans et contre une direction de soulèvement (A), dans lequel la surface de butée (26) forme un angle, de préférence un angle droit, avec l'axe de mouvement (K), dans lequel le corps de soupape (12) présentent une jupe (44) qui entoure la surface d'appui (26) radialement à l'extérieur par rapport à l'axe de mouvement (K) et qui, à partir d'une section de corps de soupape (28) présentant la surface d'appui, fait saillie axialement de la section de corps de soupape (28) par rapport à l'axe de mouvement (K) et fait ainsi saillie axialement au-delà de la surface d'appui (26),
**caractérisé en ce qu'**une distance (h), à mesurer parallèlement à la direction de soulèvement (A), d'un bord de jupe (48) éloigné de la surface de butée (26) par rapport à la surface de butée (26) varie, au moins dans des sections circonférentielles, en fonction de la position respective dans la direction circonférentielle.
